# EUROPÄISCHE PATENTSCHRIFT

(11) **EP 2 289 575 B1**
(45) Veröffentlichungstag und Bekanntmachung des Hinweises auf die Patenterteilung: **05.07.2017**
(21) Anmeldenummer: 10168721.8
(22) Anmeldetag: 07.07.2010
(51) Int. Cl.: A61L 31/14

(54) **Medizinisches Implantat enthaltend eine antioxidative Substanz**
Medical implant containing an antioxidative substance
Implant médical contenant une substance anti-oxydante

(30) Priorität: 06.08.2009 US 231688 P
(43) Veröffentlichungstag der Anmeldung: 02.03.2011
(73) Patentinhaber: Biotronik VI Patent AG, 6341 Baar (CH)
(72) Erfinder: Borck, Alexander, 91086, Aurachtal (DE)
(74) Vertreter: Randoll, Sören

(56) Entgegenhaltungen:
- EP-A1- 1 779 877
- EP-A1- 1 872 809
- WO-A2-2008/034031
- US-A1- 2004 224 003

## Beschreibung

Die Erfindung betrifft ein medizinisches Implantat, wobei eine antioxidative Substanz in den Grundkörper eingearbeitet und/oder auf diesem beschichtet vorliegt

Medizinische Implantate haben in vielfältiger Ausführungsform Anwendung in der modernen Medizintechnik gefunden. Sie dienen beispielsweise der Unterstützung von Gefäßen, Hohlorganen und Gangsystemen (endovaskuläre Implantate), zur Befestigung und temporären Fixierung von Gewebeimplantaten und Gewebstransplantationen, aber auch zu orthopädischen Zwecken, z. B. als Nagel, Platte oder Schraube.

So hat sich zum Beispiel die Implantation von Stents als eine der wirkungsvollsten therapeutischen Maßnahmen bei der Behandlung von Gefäßerkrankungen etabliert. Eine der häufigsten Todesursachen in der entwickelten Welt sind kardiovaskuläre Erkrankungen, wobei Koronarerkrankungen von höchster Bedeutung sind. Zur Behandlung dieser Erkrankungen werden intravaskulär Gefäßprothesen, wie zum Beispiel Ballons oder Stents, in das betroffene Blutgefäß eines Patienten eingebracht und gegebenenfalls implantiert, um dieses aufzuweiten und offen zu halten.

Das Implantat besitzt einen Grundkörper aus einem Implantatwerkstoff. Ein Implantatwerkstoff ist ein nicht lebendes Material, das für eine Anwendung in der Medizin eingesetzt wird und mit biologischen Systemen in Wechselwirkung tritt. Grundvoraussetzungen für den Einsatz eines Werkstoffes als Implantatwerkstoff, der bei bestimmungsgemäßer Verwendung mit der Körperumgebung in Kontakt steht, ist dessen Körperverträglichkeit (Biokompatibilität). Unter Biokompatibilität wird die Fähigkeit eines Werkstoffes verstanden, in einer spezifischen Anwendung eine angemessene Gewebereaktion hervorzurufen. Dies beinhaltet eine Anpassung der chemischen, physikalischen, biologischen und morphologischen Oberflächeneigenschaften eines Implantates an das Empfängergewebe mit dem Ziel einer klinisch erwünschten Wechselwirkung. Die Biokompatibilität des Implantatswerkstoffs ist weiterhin abhängig vom zeitlichen Ablauf der Reaktion des Biosystems, in das implantiert wird. So treten relativ kurzfristig Reizungen und Entzündungen auf, die zu Gewebeveränderungen führen können. Biologische Systeme reagieren demnach in Abhängigkeit von den Eigenschaften des Implantatswerkstoffs in verschiedener Weise. Entsprechend der Reaktion des Biosystems können die Implantatswerkstoffe in bioaktive, bioinerte und degradierbare/resorbierbare Werkstoffe unterteilt werden.

Allerdings kann es aufgrund des intravaskulären Eingriffs zu einer erhöhten Thrombusbildung sowie einer erhöhten Proliferation von glatten Muskelzellen kommen, was zu einem erneuten Gefäßverschluss (Restenose) führen kann. Überschießende Proliferation von Narbengewebe führt bei ca. 30 - 40% aller unbeschichteten Stents nach längerer Zeit zu einer Restenose.

Um die Risikofaktoren einer Restenose zu verhindern, wurde eine Vielzahl an Beschichtungen für Stents entwickelt, die eine erhöhte Hämokompatibilität bieten sollen. Seit längerer Zeit werden bspw. in die Beschichtung der Stents antikoagulierende, antimikrobielle, antiinflammatorische und antiproliferative Agenzien einzeln oder in Kombination eingesetzt. Diese pharmazeutisch aktiven Substanzen (Wirkstoffe) sollen aus dem Beschichtungsmaterial des Stents derart freigesetzt werden, dass sie Entzündungen des umliegenden Gewebes, überschießendes Wachstum der glatten Muskelzellen oder das Verklumpen von Blut verhindern. Allerdings besteht bei diesen beschichteten Implantate das Problem, dass sie nach ihrer Produktion bis zum Implantationszeitpunkt in der Regel eine geringe Haltbarkeit aufweisen, d. h. sie können nur über einen kurzen Zeitraum gelagert werden, oder benötigen zudem besondere Lagerungsbedingungen, wie beispielsweise eine Lagerung der Produkte bei 4°C. Dies führt zu einem erhöhten Ausschuss an gefertigten Produkten und somit zu einem erhöhten wirtschaftlichen Verlust sowie zu erhöhten Kosten hinsichtlich des Energieverbrauchs. Insbesondere medizinische Implantate aus polymeren Materialien oder mit Beschichtungen aus polymeren Materialien, ggf. beladen mit Wirkstoffen, müssen hinsichtlich ihrer Haltbarkeit und Lagerstabilität verbessert werden.

Weiterhin haben viele dieser wirkstoffbeladenen beschichteten Stents den Nachteil, dass die Wirkstoffe zu langsam an den umliegenden Implantationsort abgegeben werden sowie deren Bioverfügbarkeit aus den jeweiligen Beschichtungsmaterialien zu gering ist. Weiterhin ist bei der Einstellung der Dosierung des freizusetzenden Wirkstoffs limitierend, dass die Menge des auf die Außenseite der Stents aufzubringenden Wirkstoffs stark begrenzt ist, da die für die Aufbringung bereitstehenden Flächen am Stent sehr klein sind. Bei Stents aus biokorrodierbaren Magnesiumlegierungen kann zusätzlich das Problem auftreten, dass das durch Korrosion des Werkstoffs entstehende stark alkalische Milieu das Resorptionsverhalten des aufzunehmenden Wirkstoffs negativ beeinflusst. So werden Wirkstoffe teils als Hydrochloride eingesetzt, wenn die Löslichkeit des Wirkstoffs sonst zu gering ist. Derartige Hydrochloride werden jedoch im entstehenden stark alkalischen Milieu wieder in die schlecht löslichen deprotonierten Wirkstoffe überführt.

Ein Problem beim Einsatz von biokorrodierbaren Implantaten, die ganz oder in Teilen aus einem metallischen Werkstoff bestehen, liegt weiterhin darin, dass die Abbauprodukte, die bei der Korrosion des Implantats entstehen und freigesetzt werden, oft einen merklichen Einfluss auf den lokalen pH-Wert haben und zu ungewünschten Gewebsreaktionen führen können. Zudem weisen diese biokorrodierbaren Implantate auf Grund ihrer erhöhten Korrosionsrate häufig eine für den gewünschten Verwendungszweck und Implantationsort zu kurze Implantatintegrität auf. Insbesondere beim Abbau von Mg-haltigen biokorrodierbaren Implantatwerkstoffen kann es zu einem Anstieg des pH-Wertes in der unmittelbaren Umgebung kommen. Dieser Anstieg des pH-Werts kann zu einem Phänomen führen, dass unter dem Begriff Alkalose zusammengefasst wird. Der lokale pH-Wert-Anstieg führt dabei zu einem Ungleichgewicht der Ladungsverteilung in den das Gefäß umgebenden glatten Muskelzellen, was dazu führen kann, dass es zu einer lokalen Erhöhung des Muskeltonus im Bereich des Implantats kommt. Dieser erhöhte Druck auf das Implantat kann zum vorzeitigen Verlust der Implantatintegrität führen. Ist das Implantat z. B. ein Stent, so kann es im Zuge einer solchen Vasokonstriktion im Gefäßbereich um den Stent zu einer Restenose oder einer Gefäßlumenbeeinträchtigung kommen.

In EP 1779877 A1 werden Implantate offenbart. In WO 2008034031 A2 werden abbaubare Endoprothesen offenbart. In EP 1872809 A1 werden beschichtete Implantate beschrieben. In US 2004/0224003 A1 werden Beschichtungen für medizinische Geräte offenbart.

Der vorliegenden Erfindung liegt die Aufgabe zugrunde, eines oder mehrere der zuvor geschilderten Probleme zu lösen oder zumindest zu mindern.

Insbesondere soll die Lagerstabilität der beschichteten medizinischen Implantate sowie die Bioverfügbarkeit und Resorption pharmazeutisch aktiver Substanzen verbessert werden, wenn sie Bestandteil einer Beschichtung eines Implantats sind. Weiterhin soll die Korrosionsbeständigkeit von biokorrodierbaren medizinischen Implantaten optimiert werden.

Die Aufgabe wird durch Bereitstellung eines medizinischen Implantats gelöst, wobei der Grundkörper mindestens die antioxidative Substanz Squalen aufweist und Squalen in Mengen von 0,4 µg bis 2000 µg im Grundkörper eingearbeitet und/öder auf diesem beschichtet vorliegt.

Es hat sich überraschenderweise gezeigt, dass eine Zugabe von geringen Mengen an Squalen die Gewebsaufnahme sowie die Bioverfügbarkeit pharmazeutisch aktiver Substanzen verbessert ohne die sonstigen mechanischen Eigenschaften des Implantats nachteilig zu beeinflussen. Die mechanischen Eigenschaften einiger Implantatwerkstoffe konnten sogar verbessert werden. Weiterhin hat sich gezeigt, dass bereits geringe Mengen an Squalen die Haltbarkeit und die Lagerung medizinischer Implantate deutlich verbessern. Zudem reichen geringe Mengen an Squalen aus, die Korrosion eines biokorrodierbaren Implantats merklich zu verzögern.

Squalen, auch 2,6,10,15,19,23-Hexamethyl-2,6,10,14,18,22-tetracosahexaen, Spinacen oder Supraen genannt, gehört zu der Klasse der Isoprenoiden und wird als Muttersubstanz der Triterpene (C30) angesehen und spielt eine wesentliche Rolle bei der Biosynthese von lebenswichtigen Substanzen höherer Organismen. Diese symmetrisch gebaute, aliphatische Verbindung ist vor allem Ausgangssubstanz für die Bildung der Steroide, zu denen wichtige Verbindungen wie Sterole, Gallensäuren, Steroidhormone, Vitamine der D-Gruppe, Saponine und Herzglycoside gehören. Bei der Biosynthese von beispielsweise Cholesterin wird Squalen intermediär durch reduktive Dimerisierung von Farnesyldiphosphat erhalten, welches anschließend über eine Squalenoxid-Zwischenstufe zu Lanosterin weiterreagiert, eine Vorstufe des Cholesterins.

Ein wesentlicher Vorteil von Squalen ist seine deutlich verbesserte Körperverträglichkeit im Hinblick auf Toxizität im Vergleich zu anderen Isoprenoiden oder Isoprenoid-Derivaten sowie seine verbessert Anreicherung im Körper. So wirken beispielsweise Lycopene und Ubichinon in Konzentrationen von 10 µMol/L bereits toxisch. Dahingegen wirkt Squalen auch in Konzentrationen von 100 µMol/L nicht toxisch.

Die Verwendung von Squalen als Hilfsstoff auf medizinischen Implantaten hat zwei entscheidende Vorteile.

Bei Wirkstoff freisetzenden Implantaten kann die Gesamtmenge an Wirkstoffbeladung reduziert werden, ohne dass die Wirkpotenz des Implantates vermindert ist. Die lipophilen Eigenschaften des Squalens sind dafür verantwortlich. Die Wirkstoffverteilung bzw. auch die Wirkstoffpenetration wird beeinflusst. Das Squalen zieht wie ein Film auf die Gefäßinnenseite auf, der im Squalenfilm gelöste bzw. aus dem Implantat eingewanderte Wirkstoff wird dadurch länger am Implantationsort fixiert und hat länger Zeit, in das gewünschte Zielgewebe zu gelangen. Die Aufnahme in die Zellen wird verbessert und damit die Verfügbarkeit erhöht. Dabei ist nicht entscheidend, ob der Wirkstoff aus Löchern oder Kavitäten am Implantat stammt oder aus einer polymeren Matrix freigesetzt wird.

Bei Polymeren Matrices hat der Hilfsstoff zusätzliche sehr vorteilhafte Eigenschaften. Die Zugabe von Squalen macht Polymere wie beispielsweise PLLA weicher und flexibler. Durch die Modifikation der mechanischen Eigenschaften verbessert sich das gesamte Implantat. Sichtbar wird das am Beispiel des Koronarstents. Polymere Beschichtungen können bei plastischer Verformung Abplatzen oder Risse (Microcracks) bekommen. Abplatzungen sind besonders bedenklich, da hier die Gefahr einer Thrombose gegeben ist. Risse in der Wirkstoff-tragenden Polymerschicht verändern das Freisetzungsverhalten, die *release* Kinetik. Damit kann das therapeutische Fenster verlassen werden bzw. die Wirkstoffabgabe bleibt durch eine zeitweise Überdosierung ungenutzt.

Neben den Stents, die für eine längere Wirkstofffreisetzung am Implantationsort geeignet sind, gibt es die Möglichkeit über Ballons Wirkstoff kurzfristig abzugeben. Hier eignet sich der Hilfsstoff wie oben beschrieben zur Verbesserung der Gewebspenetration, was an den lipophilen Eigenschaften des Moleküls liegt. Zusätzlich kann durch die Zugabe von Squalen im unteren Prozentbereich die Darreichungsform optimiert werden. Viele Wirkstoffe wie Beilspielsweise das Paclitaxel kristallisieren auf dem Ballonmaterial. Das hat folgende Nachteile:
- Mangelnde Haftung der Kristalle auf dem Ballonmaterial
- Verlust von Wirkstoff durch mechanische Einflüsse
- Verzögerung der Auflösungsgeschwindigkeit

Durch Verwendung des Hilfsstoffs Squalen kann die Kristallisation verhindert oder zumindest gehemmt werden. Die Wirkstoff-Hilfsstoff Mischung kann in Form einer pastösen Masse aufgetragen werden, was die genannten Nachteile vermeidet.

Das Anwendungsspektrum von Squalen kann darüber hinaus optimiert werden. Squalen neigt an der Luft zur Eigenpolymerisation. Durch die natürliche oder künstlich herbeigeführte Polymerisation des Hilfsstoffs können Materialeigenschaften noch besser angepasst werden. Die polymere Form des Hilfsstoffs hat geringere Diffusionseigenschaften und bleibt lokal z.B. als Weichmacher länger verfügbar.

Bei Wirkstoff-tragenden Implantaten konnte gezeigt werden, dass trotz beschleunigter Alterung die Lagerstabilität verbessert wird. Squalen wirkt als Antioxidanz bzw. als Radikalfänger. Durch die Zugabe des Hilfsstoffs in Mengen von weniger als 1 % kann die Lagerzeit des mit Wirkstoff beladenen Implantates um 3 bis 6 Monate, im Vergleich zu Proben ohne Antioxidanz, verlängert werden, ohne dass Abbauprodukte über die HPLC Analytik nachgewiesen werden können.

In einer bevorzugten Ausführungsform weist das medizinische Implantat einen Grundkörper auf, wobei Squalen in Mengen von 0,4 µg bis 2000 µg im Grundkörper eingearbeitet und/oder auf diesem beschichtet vorliegt. Besonders bevorzugt weist das medizinische Implantat einen Grundkörper auf, wobei Squalen in Mengen von 4 µg bis 400 µg im Grundkörper eingearbeitet und/oder auf diesem beschichtet vorliegt. Insbesondere bevorzugt weist das medizinische Implantat einen Grundkörper auf, wobei Squalen in Mengen von 40 µg bis 100 µg im Grundkörper eingearbeitet und/oder auf diesem beschichtet vorliegt.

Medizinische Implantate innerhalb des Schutzumfangs der vorliegenden Erfindungen beinhalten beliebige medizinische Vorrichtungen, welche benutzt werden, wenigstens teilweise, um in den Körper eines Patienten eingebracht zu werden. Beispiele beinhalten implantierbare Vorrichtungen Herzschrittmacher, Katheter, Nadelinjektionskatheter, Blutgerinnselfilter, vaskuläre Transplantate, Ballons, Stenttransplantate, Gallenstents, Darmstents, Bronchiallungenstents, Speiseröhrenstents, Harnleiterstents, Aneurysmenausfüllende Spulen und andere Spulenvorrichtungen, transmyokardiale Revaskularisationsvorrichtungen, perkutane myokardiale Revaskularisationsvorrichtungen. Weiterhin können beliebige natürliche und/oder künstliche Medizinprodukte eingesetzt werden, beispielsweise Prothesen, Organe, Gefäße, Aorten, Herzklappen, Schläuche, Organersatzteile, Implantate, Fasern, Hohlfasern, Membranen, Konserven, Blutbehältern, Titerplatten, Adsorbermedien, Dialysatoren, Verbindungsstücke, Sensoren, Ventile, Endoskope, Filter, Pumpenkammern sowie andere Medizinprodukte, welche hämokompatible Eigenschaften aufweisen sollen. Der Begriff Medizinprodukte ist weit zu fassen und bezeichnet insbesöndere solche Produkte, die kurzzeitig (z. B. Endoskope) oder dauerhaft (z. B. Stents) mit Blut in Kontakt kommen.

Besonders bevorzugte medizinische Implantate sind Katheter (Ballonkatheter) und Stents.

Stents herkömmlicher Bauart weisen eine filigrane Stützstruktur aus metallischen Streben auf, die zur Einbringung in den Körper zunächst in einem nicht-expandierten Zustand vorliegen und die am Ort der Applikation dann in einen expandierten Zustand aufgeweitet werden. Der Stent kann vor oder nach dem Crimpen auf einen Ballon beschichtet werden.

### Biokorrodierbare (Degradierbare) Implantatgrundkörper, vorzugsweise biokorrodierbare Stentgrundkörper:

Im Sinne der vorliegenden Erfindung bedeutet "biokorrodierbare Implantat(grundkörper)", bevorzugt "biokorrodierbarer Stent(grundkörper)", dass der Grundkörper in physiologischer Umgebung, insbesondere im Gefäßsystem eines menschlichen oder tierischen Organismus degradiert, d. h., so abgebaut wird, dass der Stent seine Integrität verliert. Vorzugsweise degradieren erfindungsgemäß biokorrodierbaren Grundkörper erst dann, wenn die Funktionen des Implantats nicht mehr physiologisch sinnvoll bzw. notwendig sind. Bei biokorrodierbaren Stents bedeutet das, dass der Stent vorzugsweise erst dann degradiert oder seine Integrität verliert, wenn das traumatisierte Gewebe des Gefäßes verheilt ist und der Stent somit nicht länger im Gefäßlumen verbleiben muss.

In einer bevorzugten erfindungsgemäßen Ausgestaltung umfasst oder besteht der biokorrodierbare Grundkörper (Werkstoff) bevorzugt aus einem metallischen Werkstoff, der eine biokorrodierbare Legierung darstellt, wobei die Hauptkomponente der Legierung ausgewählt wird aus der Gruppe Magnesium, Eisen, Zink. Mangan und/oder Wolfram; insbesondere wird für einen biokorrodierbaren metallischen Werkstoff eine Magnesiumlegierung bevorzugt.

Die Legierung, insbesondere umfassend Magnesium, Eisen, Zink und/oder Wolfram, ist so in ihrer Zusammensetzung zu wählen, dass sie biokorrodierbar ist. Als biokorrodierbar im Sinne der vorliegenden Erfindung werden Legierungen bezeichnet, bei denen in physiologischer Umgebung ein Abbau (Degradierung) stattfindet, der letztendlich dazu führt, dass der gesamte Stent oder der aus dem Werkstoff gebildete Teil des Stents seine mechanische Integrität verliert. Unter Legierung wird vorliegend ein metallisches Gefüge verstanden, dessen Hauptkomponente Magnesium, Eisen, Zink und/oder Wolfram ist. Hauptkomponente ist die Legierungskomponente, deren Gewichtsanteil an der Legierung am höchsten ist. Ein Anteil der Hauptkomponente beträgt vorzugsweise mehr als 50 Gew.%, weiter bevorzugt mehr als 70 Gew.%. Eine Magnesium-Legierung ist hiervon besonders bevorzugt.

Ist der erfindungsgemäße Werkstoff eine Magnesium-Legierung, so enthält diese vorzugsweise Yttrium und weitere Seltenerdmetalle, da sich eine derartige Legierung aufgrund ihrer physiko-chemischen Eigenschaften und ihrer hohen Biokompatibilität, insbesondere auch ihrer Abbauprodukte, auszeichnet.

Besonders bevorzugt werde die in EP 1 419 793 B1 beschriebenen Yttrium (W) und Seltene Erden (E) enthaltenden biokorrodierbaren Magnesiumlegierungen (WE43 & WE54 von Magnesium Elektron) mit einem Anteil von Magnesium >90 Gew., Yttrium 3,7 - 5,5 Gew.%, Seltenerdmetallen 1,5 - 4,4 Gew.% und Rest < 1 Gew.%, zur Herstellung von Implantaten (Stents) eingesetzt.

Diese Magnesiumlegierungen bestätigen bereits experimentell und in ersten klinischen Versuchen ihre besondere Eignung, d. h. sie zeigen eine hohe Biokompatibilität, günstige Verarbeitungseigenschaften, gute mechanische Kennwerte und ein für die Einsatzzwecke adäquates Korrosionsverhalten.

Unter der Sammelbezeichnung "Seltene Erden" SE werden in der vorliegenden Anmeldung Scandium (21) und Yttrium (39) sowie die "Leichten Seltenen Erden" LSE Lanthan (57), Cer (58), Neodym (60), und Promethium (61) und die "Schweren Seltenen Erden" SSE Samarium (62), Europium (63), Gadolinium (64), Terbium (65), Dysprosium (66), Holmium (67), Erbium (68), Thulium (69), Ytterbium (70) und Lutetium (71) verstanden.

Erfindungsgemäße Implantatgrundkörper, bevorzugt Stentgrundkörper, können gemäß einer weiteren alternativen erfindungsgemäßen Ausgestaltung degradierbare Polymere umfassen oder daraus bestehen, vorzugsweise ausgewählt aus der Gruppe bestehend aus Polydioxanon; Polyglycolid; Polycaprolacton; Polyhydroxyvaleriansäure; Polyhydroxybuttersäure; Polylactide wie Poly(L-lactid), Poly(D-lactid), Poly(D,L-lactid) und Blends sowie Copoylmere, Poly(L-lactid-co-glycolid), Poly(D,L-lactid-co-glycolid), Poly(L-lactid-co-D-L-lactid), Poly(L-lactid-co-trimethylencarbonat) und Tri-blockcopolymere; Polysaccharide wie Chitosan, Levan, Hyaluronsäure, Heparin, Dextran und Cellulose.

### Dauerhafter Implantatgrundkörper, vorzugsweise dauerhafter Stentgrundkörper:

Im Gegensatz zum biokorrodierbaren, degradierbaren Grundkörper wird der "dauerhafte Implantatgrundkörper", vorzugsweise der "dauerhafte Stentgrundkörper" in physiologischer Umgebung im menschlichen oder tierischen Organismus im Wesentlichen nicht abgebaut, so dass er seine Integrität nicht verliert.

In einer weiteren erfindungsgemäßen bevorzugten Ausgestaltung umfasst oder besteht der Grundkörper eines dauerhaften Implantats, vorzugsweise eines dauerhaften Stents, aus einem Formgedächtnis-Material, vorzugsweise aus einem oder mehreren Materialien ausgewählt aus der Gruppe der Nickel-Titan-Legierungen und Kupfer-Zink-Aluminium-Legierungen, weiter bevorzugt Nitinol.

In einer besonders bevorzugten erfindungsgemäßen Ausgestaltung umfasst oder besteht der Grundkörper eines dauerhaften Implantats, vorzugsweise eines dauerhaften Stents aus Edelstahl, vorzugsweise aus Cr-Ni-Fe-Stahl, hier bevorzugt die Legierung 316L, einem Co-Cr-Stahl oder PERSE, einem Platin-angereichertem Edelstahl.

In einer weiteren bevorzugten Ausgestaltung kann der Implantatgrundkörper, vorzugsweise Stentgrundkörper zusätzlich Kunststoff, vorzugsweise Polyetherurethan, und/oder Keramik und/oder weitere Polymerbeschichtungen umfassen.

Katheter im Rahmen der vorliegenden Erfindung sind Röhrchen oder Schläuche verschiedenen Durchmessers, die in den jeweiligen zu behandelnden Körperhohlraum eingeführt werden können. So genannte Ballonkatheter werden vor allem in der Angioplastie zur Erweiterung oder Wiedereröffnung eines Gefäßes eingesetzt. Ein Führungsdraht wird zuerst in das zu behandelnde Gefäß eingeschoben und anschließend wird der Ballonkatheter, der aus einem Schlauch besteht, der in einem vorgegebenen Bereich entlang des Schlauchs einen nicht dilatierten Ballon aufweist, entlang des Führungsdrahts bis zu der zu behandelnden Stelle des Gefäßes vorgeschoben, so dass der Ballon im Bereich der zu behandelnden Stelle des Gefäßes, die z. B. eine Stenose aufweist, platziert ist. Danach wird der Ballon dilatiert, d. h. entfaltet und/oder aufgedehnt, so dass die zu behandelnde Stelle wiedereröffnet oder erweitert wird. Schließlich wird der Ballon wieder entleert und entlang des Führungsdrahts wieder aus dem Gefäß entfernt. Gleichzeitig oder anschließend wird auch der Führungsdraht aus dem Gefäß zurückgezogen. Durch die Erweiterung bzw. Wiedereröffnung des Gefäßes wird der Strom der Körperflüssigkeit in dem Gefäß nicht mehr oder nicht mehr in dem zuvor vorhandenen Maße behindert.

Die erfindungsgemäßen Ballonkatheter bestehen bevorzugt aus einem Material enthaltend ein Polymer, ein Copolymer und/oder einer Mischung aus mehreren Polymeren und/oder Copolymeren. Grundsätzlich können hier Polymere oder Copolymere eingesetzt werden, die die nötige Stabilität und Biegsamkeit für den Einsatz in einem Katheter aufweisen. Dem Fachmann sind solche Polymere und Copolymere bzw. Mischungen daraus aus dem Stand der Technik bekannt. Die Verträglichkeit sowie andere Eigenschaften des Polymers kann der Fachmann in einfachen Routineversuchen ohne Schwierigkeiten bestimmen. Bevorzugt werden Polymere und/oder Copolymere verwendet, die mindestens ein sich wiederholendes Monomer mit mindestens einer Amidgruppe aufweisen. Insbesondere können Polyamide, Polyamidcopolymere oder polyamid-haltige Copolymere eingesetzt werden. Dabei sind Polyamide mit mindestens 6 C-Atomen pro Monomer bevorzugt, besonders bevorzugt mit mehr als 10, ganz besonders bevorzugt mit 12 C-Atomen. Insbesondere können Polyamid-12 oder Polyamid-12-Copolymere, wie z. B. Polyamid-12/6 eingesetzt werden. Auch Polyether-Blockamide (z. B. solche unter dem Markennamen PEBAX®) können verwendet werden. Des Weiteren können bevorzugt Polymere oder Copolymere der Gruppe der Polyester, Copolyester und/oder Polyester-Elastomere eingesetzt werden.

Derartige Ballonkatheter können auch dazu verwendet werden, intraluminale Endoprothesen (Stents) an eine zu behandelnde Stelle in einem Körperhohlraum einzubringen.

In einer bevorzugten Ausführungsform wird das Squalen in Mengen von 0,4 µg bis 2000 µg in den Implantatgrundkörper eingearbeitet. Dies ist insbesondere bevorzugt bei Implantaten mit einem Werkstoff ausgewählt aus der Gruppe der biokorrodierbaren und dauerhaften Polymeren, vorzugsweise 4 µg bis 400 µg. Es hat sich insbesondere gezeigt, dass die polymere Matrix durch das Squalen deutlich stabilisiert wird und so eine längere Lagerstabilität dieser Implantate erreicht werden kann. Durch den Zusatz der antioxidativen Substanz Squalen können Radikale, die in die Polymere Matrix eindringen oder dort entstehen, sofort neutralisiert werden.

In einer weiteren bevorzugten Ausführungsform liegt das Squalen in Mengen von 40 µg bis 100 µg als Beschichtung auf dem Grundkörper eines medizinischen Implantats vor. Eine Beschichtung im Sinne der Erfindung ist eine zumindest abschnittsweise Auftragung der Komponenten auf den Grundkörper des Implantats. Vorzugsweise wird die gesamte Oberfläche des Grundkörpers des Implantats, insbesondere ein Stent oder ein Ballonkatheter, von der Beschichtung bedeckt. Eine Schichtdicke liegt vorzugsweise im Bereich von 1 µm bis 100 µm, vorzugsweise besonders bevorzugt 3 µm bis 15 µm. Die Beschichtung besteht bevorzugt aus der antioxidativen Substanz Squalen.

In einer weiteren Ausführungsform enthält die Beschichtung zumindest eine antioxidative Substanz sowie gegebenenfalls eine oder mehrere pharmazeutisch aktive Substanzen. Bevorzugt liegt das Squalen in Mengen von 1% bis 2% als Beschichtung auf dem Grundkörper eines medizinischen Implantats vor.

Alternativ kann die Beschichtung, enthaltend oder bestehend aus der mindestens einen antioxidativen Substanz Squalen, als Kavitätenfüllung vorliegen oder Bestandteil einer Kavitätenfüllung sein. Das Implantat, insbesondere der Stent, weist zu diesem Zweck eine oder mehrere Kavitäten auf. Kavitäten befinden sich beispielsweise an der Oberfläche des Implantats und lassen sich beispielsweise durch Laserablation in Nano- bis Mikrometerdimensionen erstellen. Bei Implantaten, insbesondere Stents, mit einem biodegradierbaren Grundkörper kann eine Kavität auch im Innern des Grundkörpers angeordnet sein, so dass die Freisetzung des Material erst nach Freilegung erfolgt. Der Fachmann kann sich bei der Ausgestaltung der Kavität an dem im Stand der Technik beschriebenen Systemen orientieren. Der Begriff "Kavität" umfasst dabei z. B. Löcher und Vertiefungen.

Die Beschichtung kann zusätzlich zur antioxidativen Substanz eine oder mehrere pharmazeutisch aktive Substanz aufnehmen. Alternativ können die genannten Substanzen Bestandteil einer Kavitätenfüllung sein.

Eine pharmazeutisch aktive Substanz im Rahmen der vorliegenden Erfindung sind Substanzen (Wirkstoffe) ausgewählt aus der Gruppe umfassend Antiphlogistika, vorzugsweise Dexamethason, Methylprednisolon und Diclophenac; Cytostatika, vorzugsweise Paclitaxel, Colchicin, Actinomycin D und Methotrexat; Immunsuppressiva, vorzugsweise Limus-Verbindungen, weiter bevorzugt Sirolimus (Rapamycin), Zotarolimus (Abt-578), Tacrolimus (FK-506), Everolimus, Biolimus, insbesondere Biolimus A9 und Pimecrolimus, Cyclosporin A und Mycophenolsäure; Thrombozytenaggregationshemmer, vorzugsweise Abciximab und Iloprost; Statinen, vorzugsweise Simvastatin, Mevastatin, Atorvastatin, Lovastatin, Pitavastatin, Pravastatin und Fluvastatin; Estrogenen, vorzugsweise 17b-Estradiol, Daizein und Genistein; Lipidregulatören, vorzugsweise Fibrate; Immunsuppressiva; Vasodilatatoren, vorzugsweise Sartane; Calciumkanalblocker; Calcineurininhibitoren, vorzugsweise Tacrolimus; Antiinflammatorika, vorzugsweise Imidazole; Antiallergika; Oligonucleotiden, vorzugsweise Decoy-Oligodesöxynukleotid (dODN); Endothelbildner, vorzugsweise Fibrin; Steroiden; Proteinen/Peptiden; Proliferationshemmer; Analgetika und Antirheumatika; Endothelinrezeptor-Antagonisten, vorzugsweise Bosentan; Rho-Kinase Inhibitoren, vorzugsweise Fasudil; RGD-Peptiden und zyklische RGD (cRGD) (umfassend die Sequenz Arg-Gly-Asp); und organischen Gold- oder Platinverbindungen.

Die pharmazeutisch aktive Substanz ist bevorzugt in einer pharmazeutisch aktiven Konzentration von 0,2 bis 3,5 µg/mm² Stentoberfläche, weiter bevorzugt von 0,25 bis 1,45 µg/mm² Stentoberfläche enthalten.

Die Beschichtung enthält dabei die oben definierten Mengenangaben an Squalen. Durch die antioxidative Wirkung werden die pharmazeutisch aktiven Substanzen in der Matrix konserviert, die sonst einem Abbau unterliegen würden. Dieser Abbau ist der Grund, warum herkömmliche Wirkstoff-freisetzende Implantate mit relativ kurzen Haltbarkeitsdaten von 6 Monaten bis zu einem Jahr versehen werden. Die antioxidative Wirkung erhöht die Haltbarkeit um bis zu 3-6 Monate.

Durch die Verbesserung der mechanischen Eigenschaften wie geringere Sprödigkeit werden Risse und Abplatzungen vermindert, dadurch kommt es zu keiner übermäßigen Wirkstofffreisetzung, die durch höhere Beladungen ausgeglichen respektive überkompensiert werden muss.

Die durch die sehr lipophile Substanz Squalen erhöhte Permeabilität des Wirkstoffs trägt zusätzlich zur Einsparung bzw. Verminderung der Beladung der pharmazeutisch aktiven Substanz auf dem Implantat bei, ohne dass dadurch die Wirksamkeit herabgesetzt wird.

Die Beschichtung kann eine die antioxidative Substanz und gegebenenfalls die eine oder mehrere pharmazeutisch aktive Substanzen aufnehmende biostabile und/oder bioabbaubare Polymermatrix beinhalten. Alternativ können die genannten Substanzen Bestandteil einer Kavitätenfüllung sein. Die antioxidative und die pharmazeutisch aktive Substanz können räumlich voneinander getrennt, ggf. auch in verschiedenen Matrices, in der Beschichtung vorliegen.

Eine biostabile und/oder bioabbaubare Polymermatrix oder -schicht im Sinne der Erfindung ist eine zumindest abschnittsweise Auftragung der Komponenten der Beschichtung auf das medizinische Implantat. Vorzugsweise wird die gesamte Oberfläche des medizinischen Implantats von der Beschichtung bedeckt. Die Schichtdicke liegt vorzugsweise im Bereich von 2 µm bis 60 µm, besonders bevorzugt von 10 µm bis 30 µm.

Die Beschichtung kann direkt auf das medizinische Implantat aufgetragen werden. Die Verarbeitung kann nach Standardverfahren für die Beschichtung erfolgen. Es können einschichtige, aber auch mehrschichtige Systeme (zum Beispiel so genannte base coat-, drug coat- oder top coat-Schichten) erstellt werden. Die Beschichtung kann unmittelbar auf dem Grundkörper des Implantats aufgebracht sein oder es sind weitere Schichten dazwischen vorgesehen, die beispielsweise der Haftvermittlung dienen.

Die biostabile und/oder bioabbaubare Polymerschicht im Rahmen der vorliegenden Erfindung ist zusammengesetzt aus Polymeren ausgewählt aus der Gruppe bestehend aus nichtresorbierbaren, permanenten Polymeren und/oder resorbierbaren bioabbaubaren Polymeren.

Besonders bevorzugt ist die biostabile und/oder bioabbaubare Polymerschicht zusammengesetzt aus Polymeren ausgewählt aus der Gruppe Polyolefine, Polyetherketone, Polyether, Polyvinylalkohole, Polyvinylhalogenide, Polyvinylester, Polyacrylate, Polyhalogenolefine, Polyamide, Polyamidimide, Polysulfone, Polyester, Polyurethane, Silikone, Polyphosphazene, Polyphenylen, Polymerschäume (aus Styrolen und Carbonaten), Polydioxanone, Polyglycolide, Polylactide, Poly-ε-caprolacton, Ethylvinylacetat, Polyethylenoxid, Polyphosphörylcholin, Polyhydroxybuttersäuren, Lipide, Polysaccharide, Proteine, Polypeptide sowie Copolymere, Blends und Derivate dieser Verbindungen.

Ganz besonders bevorzugt ist die biostabile und/oder bioabbaubare Polymerschicht zusammengesetzt aus Polymeren ausgewählt aus der Gruppe bestehend aus Polypropylen, Polyethylen, Polyisobutylen, Polybutylen, Polyetheretherketon, Polyethylenglycol, Polypropylenglycol, Polyvinylalkohole, Polyvinylchlorid, Polyvinylfluorid, Polyvinylacetat, Polyethylacrylat, Polymethylacrylat, Polytetrafluorethylen, Polychlortrifluorethylen, PA 11, PA 12, PA 46, PA 66, Polyamidimide, Polyethersulfon, Polyphenylsulfon, Polycarbonate, Polybutylenterephthalat, Polyethylenterephthalat, Elastane, Pellethane, Silikone, Polyphosphazene, Polyphenylen, Polymerschäume (aus Styrolen und Carbonaten), Polydioxanone, Polyglycolide, Poly-L-, Poly-D-, und Poly-D,L-Lactid, sowie Poly-ε-caprolacton, Ethylvinylacetat, Polyethylenoxid, Polyphosphorylcholin, Polyhydroxyvalerat, Cholesterin, Cholesterinester, Alginat, Chitosan, Levan, Hyaluronsäure, Uronide, Heparin, Dextran, Cellulose, Fibrin, Albumin, Polypeptide und Copolymere, Blends und Derivate dieser Verbindungen.

Die biostabile und/oder bioabbaubare Polymerschicht richtet sich bevorzugt nach der gewünschten Elutionsgeschwindigkeit sowie den individuellen Charakteristika der verschiedenen eingesetzten Wirkstoffe und nach der unterschiedlichen Resorptions- bzw. Degradationsgeschwindigkeit am Wirkort des Medizinproduktes.

### Ausführungsbeispiel 1: Mit Squalen beschichteter, Wirkstoff-freisetzender Stent

Ein permanenter oder wahlweise ein biokorrodierbarer Stent wird wie folgt beschichtet:
Der Stent wird von Staub und Rückständen gereinigt und in eine geeignete Stentbeschichtungsapparatur (DES Coater, Eigenentwicklung Fa. Biotronik) eingespannt. Mit Hilfe eines Airbrush Systems wird der sich drehende Stent unter konstanten Umgebungsbedingungen (Raumtemperatur; 42% Luftfeuchte) halbseitig mit PLLA enthaltend pharmakologisch aktive Substanz und Squalen als Hilfsstoff (bis 2 % bezogen auf den Feststoffgehalt der Beschichtungslösung) beschichtet. Bei einem Düsenabstand von 20 mm ist ein 18 mm langer Stent nach ca. 2 min beschichtet. Nach Erreichen der beabsichtigten Schichtmasse wird der Stent 5 Min. bei Raumtemperatur getrocknet, ehe nach Drehen des Stents und erneutem Einspannen die unbeschichtete Seite auf dieselbe Weise beschichtet wird.

### Ausführungsbeispiel 2: Mit polymerisiertem Squalen beschichteter Wirkstoff-freisetzender Stent

Abweichend von Ausführungsbeispiel 1 enthält die Sprühlösung (10 g PLLA; L210 Boehringer Ingelheim 2,51 Chloroform) folgende zusätzliche Bestandteile:
1 ml Squalen (Dichte 0,86 g/cm³)
50 µl Triethanolamin
5 µl einer Vinylpyrrolidon Lösung enthaltend 0,3 % Eosin Y

Die Bestandteile werden vor der Beschichtung gemischt und im Vorlagegefäß unter Lichtausschluss gerührt.

Der Stent wird wie in Ausführungsbeispiel 1 beschrieben beschichtet, dabei wird der Stent mit einer UV Röhre mit 360 nm belichtet. Mit Hilfe des Photoinitiators geschieht die Vernetzung des Squalens direkt am Stent. Nach 2 Min. kann der Stent gedreht und von der anderen Seite analog beschichtet werden.

## Patentansprüche

1. Medizinisches Implantat, wobei der Grundkörper mindestens die antioxidative Substanz Squalen aufweist, **dadurch gekennzeichnet, dass** Squalen in Mengen von 0,4 µg bis 2000 µg im Grundkörper eingearbeitet und/oder auf diesem beschichtet vorliegt, wobei das Implantat ein Ballonkatheter oder Stent ist.

2. Implantat nach Anspruch 1, **dadurch gekennzeichnet, dass** Squalen in Mengen von 4 µg bis 400 µg in dem Grundkörper eingearbeitet und/oder auf diesem beschichtet vorliegt.

3. Implantat nach Anspruch 1 oder 2, **dadurch gekennzeichnet, dass** der Stent einen biokorrodierbaren oder dauerhaften Grundkörper aufweist.

4. Implantat nach Anspruch 3, **dadurch gekennzeichnet, dass** der biokorrodierbare Grundkörper einen biokorrodierbaren metallischen Werkstoff umfasst oder aus daraus besteht, ausgewählt aus Legierungen aus der Gruppe Magnesium, Eisen, Zink Mangan und/oder Wolfram.

5. Implantat nach Anspruch 3, **dadurch gekennzeichnet, dass** der biokorrodierbare Grundkörper ein degradierbares Polymer umfasst oder daraus besteht ausgewählt aus der Gruppe bestehend aus Polydioxanon; Polyglycolid; Polycaprolacton; Polyhydroxyvaleriansäure; Polyhydroxybuttersäure; Polylactide wie Poly(L-lactid), Poly(D-lactid), Poly(D,L-lactid) und Blends sowie Copolymere wie Poly(L-lactid-co-glycolid), Poly(D,L-lactid-co-glycolid), Poly(L-lactid-co-D-L-lactid), Poly(L-lactid-co-trimethylencarbonat) und Tri-blockcopolymere; Polysaccharide wie Chitosan, Levan, Hyaluronsäure, Heparin, Dextran und Cellulose.

6. Implantat nach Anspruch 3, **dadurch gekennzeichnet, dass** der dauerhafte Grundkörper ein oder mehrere Materialien ausgewählt aus der Gruppe der Nickel-Titan-Legierungen, Kupfer-Zink-Aluminium-Legierungen, Edelstahl wie Cr-Ni-Fe-Stahl, 316L, Co-Cr-Stahl oder Platin-angereichertem Edelstahl umfasst oder daraus besteht.

7. Implantat nach Anspruch 3, **dadurch gekennzeichnet, dass** der dauerhafte Grundkörper ein oder mehrere Materialien ausgewählt aus der Gruppe der Kunststoffe wie Polyetherurethan und/oder Keramik umfasst oder daraus besteht.

8. Medizinisches Implantat umfassend eine Beschichtung, wobei der Grundkörper mindestens die antioxidative Substanz Squalen aufweist, **dadurch gekennzeichnet, dass** Squalen in Mengen von 0,4 µg bis 2000 µg im Grundkörper eingearbeitet und/oder auf diesem beschichtet vorliegt, wobei die Beschichtung eine bioabbaubare Polymermatrix beinhaltet.

9. Implantat nach Anspruch 8, wobei die Beschichtung ausgewählt ist aus der Gruppe Poly-L-Lactid, Poly-D-Lactid und Poly-D,L-Lactid.

10. Implantat nach einem der vorherigen Ansprüche, **dadurch gekennzeichnet, dass** das Implantat zusätzlich eine oder mehrer weitere pharmazeutisch aktive Substanzen enthält.

11. Implantat nach einem der vorherigen Ansprüche, **dadurch gekennzeichnet, dass** die antioxidative Substanz und/oder die eine oder mehreren pharmazeutisch aktiven Substanzen in eine oder mehrere gleiche oder verschiedene biostabile oder bioabbaubare Polymermatrices eingebettet sind.

## Claims

1. A medical implant, wherein the main body comprises at least the antioxidative substance squalene, **characterised in that** squalene is incorporated into the main body and/or is coated thereon in quantities of from 0.4 µg to 2,000 µg, wherein the implant is a balloon catheter or stent.

2. The implant according to claim 1, **characterised in that** squalene is incorporated in the main body and/or is coated thereon in quantities of from 4 µg to 400 µg.

3. The implant according to claim 1 or 2, **characterised in that** the stent has a biocorrodible or permanent main body.

4. The implant according to claim 3, **characterised in that** the biocorrodible main body comprises or consists of a biocorrodible metallic material selected from alloys from the group of magnesium, iron, zinc, manganese and/or tungsten.

5. The implant according to claim 3, **characterised in that** the biocorrodible main body comprises or consists of a degradable polymer selected from the group consisting of polydioxanone; polyglycolide; polycaprolactone; polyhydroxy valeric acid; polyhydroxy butyric acid; polylactides such as poly(L-lactide), poly(D-lactide), poly(D,L-lactide) and blends such as copolymers like poly(L-lactide-co-glycolide), poly(D,L-lactide-co-glycolide), poly(L-lactide-co-D,L-lactide), poly(L-lactide-co-trimethylene carbonate) and tri-block copolymers; polysaccharides such as chitosan, levan, hyaluronic acid, heparin, dextran and cellulose.

6. The implant according to claim 3, **characterised in that** the permanent main body comprises or consists of one or more materials selected from the group of nickel-titanium alloys, copper-zinc-aluminium alloys, stainless steel such as Cr-Ni-Fe steel, 316L, Co-Cr steel or platinum-enriched stainless steel.

7. The implant according to claim 3, **characterised in that** the permanent main body comprises or consists of one or more materials selected from the group of plastics such as polyurethane and/or ceramic.

8. A medical implant comprising a coating, wherein the main body comprises at least the antioxidative substance squalene, **characterised in that** squalene is incorporated in the main body and/or is coated thereon in quantities of from 0.4 µg to 2,000 µg, wherein the coating contains a biodegradable polymer matrix.

9. The implant according to claim 8, wherein the coating is selected from the group of poly(L-lactide), poly(D-lactide), and poly(D,L-lactide).

10. The implant according to any one of the preceding claims, **characterised in that** the implant additionally contains one or more further pharmaceutically active substances.

11. The implant according to any one of the preceding claims, **characterised in that** the antioxidative substance and/or the one or more pharmaceutically active substances are/is embedded in one or more of the same or different biostable or biodegradable polymer matrices.

## Revendications

1. Implant médical, dans lequel le corps de base présente au moins la substance antioxydante squalène, **caractérisé en ce que** le squalène est présent incorporé dans le corps de base et/ou y est sous forme d'un revêtement dans des quantités de 0,4 µg à 2000 µg, l'implant étant un cathéter à ballonnet ou un stent.

2. Implant selon la revendication 1, **caractérisé en ce que** du squalène est présent incorporé dans le corps de base et/ou y est sous forme d'un revêtement dans des quantités de 4 µg à 400 µg.

3. Implant selon la revendication 1 ou 2, **caractérisé en ce que** le stent présente un corps de base biocorrodable ou durable.

4. Implant selon la revendication 3, **caractérisé en ce que** le corps de base biocorrodable comprend un matériau métallique biocorrodable ou en est constitué, choisi à partir d'alliages du groupe constitué du magnésium, du fer, du zinc, du manganèse et/ou du tungstène.

5. Implant selon la revendication 3, **caractérisé en ce que** le corps de base biocorrodable comprend un polymère dégradable ou est constitué choisi dans le groupe constitué de polydioxanone ; de polyglycolide ; de polycaprolactone ; d'acide polyhydrovalérique ; d'acide polyhydroxybutyrique ; de polylactides comme le poly(L-lactide), le poly(D-lactide), le poly(D,L-lactide) et de mélanges ainsi que de copolymères comme le poly(L-lactide-co-glycolide), le poly(D,L-lactide-co-glycolide), le poly(L-lactide-co-D-L-lactide), le poly(L-lactide-co-triméthylène carbonate) et de tri-copolymères blocs ; de polysaccharides comme le chitosan, le lévane, l'acide hyaluronique, l'héparine, le dextrane et la cellulose.

6. Implant selon la revendication 3, **caractérisé en ce que** le corps de base durable comprend un ou plusieurs matériaux choisis dans le groupe constitué des alliages nickel-titane, d'alliages cuivre-zinc-aluminium, d'acier inoxydable comme l'acier Cr-Ni-Fe, le 316L, l'acier Co-Cr ou l'acier inoxydable enrichi de platine, ou en est constitué.

7. Implant selon la revendication 3, **caractérisé en ce que** le corps de base durable comprend un ou plusieurs matériaux choisis dans le groupe des matières plastiques comme le polyuréthane et/ou de céramique, ou en est constitué.

8. Implant médical comprenant un revêtement, dans lequel le corps de base présente au moins la substance antioxydante squalène, **caractérisé en ce que** le squalène est présent incorporé dans le corps de base et/ou y est sous forme d'un revêtement dans des quantités de 0,4 µg à 2000 µg, le revêtement contenant une matrice polymère biodégradable.

9. Implant selon la revendication 8, dans lequel le revêtement est choisi dans le groupe du poly-L-lactide, du poly-D-lactide et du poly-D,L-lactide.

10. Implant selon l'une des revendications précédentes, **caractérisé en ce que** l'implant contient en outre une ou plusieurs autres substances pharmaceutiquement actives.

11. Implant selon l'une des revendications précédentes, **caractérisé en ce que** la substance antioxydante et/ou la ou les substances pharmaceutiquement actives sont incorporées dans une ou plusieurs matrices polymères biostables ou biodégradables identiques ou différentes.
